# EUROPEAN PATENT APPLICATION

(11) **EP 1 681 384 A1**
(43) Date of publication of application: **19.07.2006**
(21) Application number: 05107577.8
(22) Date of filing: 17.08.2005
(51) Int. Cl.: D06F 35/00

(54) **Washing machine**

(30) Priority: 13.01.2005 KR 2005003392
(71) Applicant: Samsung Electronics Co, Ltd, Suwon-si, Gyeonggi-do (KR)
(72) Inventor: Kim, Hyun Sook, 135-1703 Whanggolmaeul Apt., Gyeonggi-do (KR); Park, Seon Woo, 922-401 Byeokjeokgol Samsung Apt., Suwon-si, Gyeonggi-do (KR); Park, Jee Hun, Suwon-si, Gyeonggi-do (KR); Oak, Seong Min, 659-7 Yandeok-2-Dong, Gueongsangnam-do (KR); Kim, Hyung Gyoon, 108-203 SsangYong Apt., Gyeonggi-do (KR)
(74) Representative: Grey, Ian Michael

(57) **Abstract**

A method of cleaning a rotatably mounted washing machine drum which receives laundry to be washed. The method includes the steps of supplying water to the drum, and rotating the drum whilst supplying heat thereto to heat the drum.

## Description

The present invention relates to a method of cleaning a rotatably mounted washing machine drum which receives laundry to be washed. The invention also relates to a washing machine.

A conventional washing machine may comprise a water tub, a drum rotatably mounted in the water tub, a water supply valve to control the flow of water supplied to the water tub, a motor to rotatably drive the drum and a pulsator. When a user places laundry in the drum and inputs a washing command, the washing machine successively performs wash, rinse and spin-drying cycles.

After a washing machine is used for a long period of time, the drum becomes dirty and/or bacteria propagates within it. Various drum disinfecting methods have been proposed with the aim of solving this problem, one example of which is disclosed in Korean Unexamined Patent Publication No. 2001-0010111.

In a conventional drum disinfecting method, a detergent and a disinfectant are placed in the drum, the pulsator is operated for a predetermined period of time to dissolve the detergent and the disinfectant. Water is supplied to the drum, and the pulsator is operated again to disinfect the drum.

A problem with the above mentioned method is that the temperature of the wash water is lower than an appropriate temperature for impurity removal and disinfection (approximately 60°C). Consequently, the drum is not cleaned and disinfected properly. If cold water is supplied to the drum, decontamination and disinfection efficiency achieved by the detergent and the disinfectant are considerably lowered.

Furthermore, a large amount of water is supplied to the drum at once so as to clean it. Therefore, the motor must be operated at a relatively low rotating speed to protect the washing motor, and therefore, only a slow water current is generated. As a result, the magnitude of the mechanical force affecting decontamination of the drum is low resulting in improper cleaning.

The present invention seeks to overcome or alleviate the problems referred to above.

A method of cleaning a rotatably mounted washing machine drum according to the present invention is characterised by the steps of supplying water to the drum, and rotating the drum whilst supplying heat thereto to heat the drum.

In a preferred embodiment, the method includes the step of supplying a predetermined initial amount of water to the drum and rotating the drum at a first predetermined speed whilst supplying heat to the drum before supplying an additional amount of water to the drum and rotating the drum at a second predetermined speed.

Preferably, the second predetermined speed is faster than the first predetermined speed.

According to the invention, there is also provided a washing machine comprising a rotatably mounted washing machine drum to receive laundry to be washed, means for supplying water to the drum and a heater characterised by a control unit configured to perform a drum cleaning cycle in which a first predetermined amount of water is supplied to the drum and the drum is rotated at a first predetermined speed before an additional predetermined amount of water is supplied to the drum and the drum is rotated at a second predetermined speed, to clean the drum.

An embodiment of the present invention will now be described, by way of example only, with reference to the accompanying drawings, in which:
Figure 1 is a sectional view of a drum-type washing machine according to an embodiment of the present invention;
Figure 2 is a block diagram of the drum washing machine shown in Figure 1;
Figure 3 is a flow chart illustrating a drum cleaning process in the drum washing machine shown in Figures 1 and 2; and
Figure 4 is a timing chart illustrating the drum cleaning process in the drum washing machine shown in Figures 1 and 2.

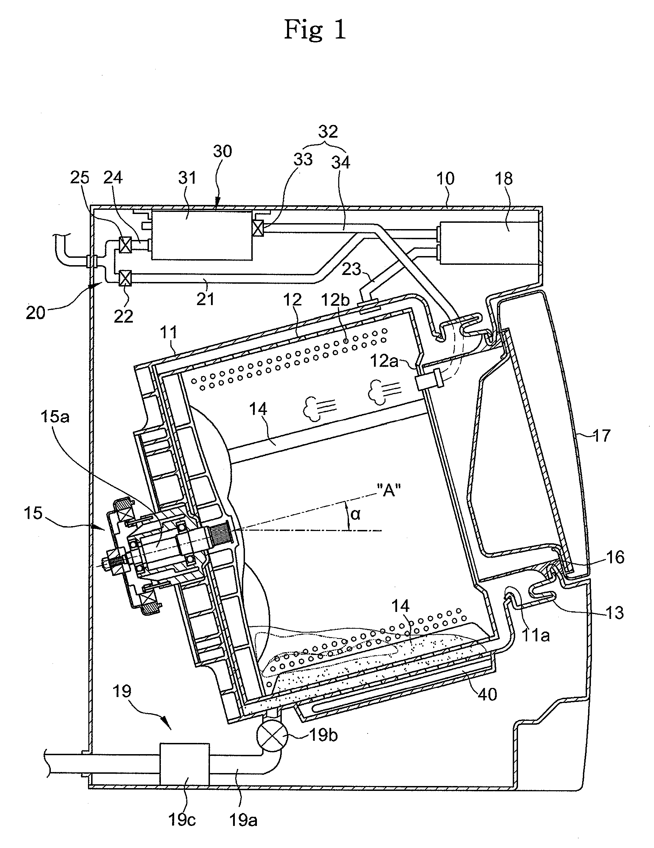

As shown in Figure 1, the drum-type washing machine comprises a drum-type water tub 11 mounted within a housing 10 to receive water, and a drum 12 rotatably mounted in the water tub 11.

The water tub 11 is disposed at a predetermined angle (α) relative to a base of the housing on which the washing machine stands so that the front part of the water tub 11, at which an inlet 11a is formed, is higher than the rear part of the water tub 11. Similarly, the drum 12 mounted in the water tub 11 at the same angle as the water tub 11 so that the front part of the drum 12, at which an inlet 12a is formed, is higher than the rear part of the drum 12. That is, the drum 12 is disposed so that the rotating centre line A of the drum 12 is at the predetermined angle (α) to the base of the washing machine while the front part of the drum 12 having the inlet 12a formed thereat faces the front upper part of the washing machine. The rear of the drum 12 is attached a rotary shaft 15a, which is rotatably supported within the rear part of the water tub 11 so that the drum 12 can rotate within the water tub 11. A plurality of through-holes 12b are formed in the cylindrical surface of the drum 12 and a plurality of lifters 14 are attached to its inner surface to lift and drop laundry as the drum 12 rotates.

A washing motor 15 to rotate the rotary shaft 15a is connected to the drum 12. Laundry may be put into the drum 12 or removed from the drum 12 via an inlet 16 corresponding to the inlet 12a of the drum 12 and the inlet 11a of the water tub 11. The inlet 16 is closed by a door 17 hingedly attached to the front part of the housing 10. A diaphragm 13 is mounted between the water tub 11 and the inlet 16 of the housing 10.

A detergent supply device 18 is mounted above the tub 11 to supply detergent to the water tub 11, a steam device 30 to supply steam to the water tub 11, a water supply device 20 to supply water to the water tub 11 and the steam device 30. A drainage device 19 is mounted under the water tub 11 and comprises a drain pipe 19a, a drain valve 19b and a drain motor 19c, to drain water from the water tub 11, and a heater 40 to heat water in the water tub 11.

The detergent supply device 18 has a detergent receiving space defined therein and is disposed at the front part of the housing 10 so that a user can easily access the detergent supply device 18 to put detergent therein. The steam device 30 comprises a steam generator 31 to generate steam and a steam supply device 32 to supply generated steam to the water tub. The steam supply device 32 comprises a steam supply pipe 34 connected between the steam generator 31 and the water tub 11, and a steam supply valve 33 mounted on the steam supply pipe 34. In the steam generator 31 is mounted a heater (not shown) to heat water supplied into the steam generator 31.

The water supply device 20 comprises a first water supply pipe 21 to supply water to the water tub 11, and a first water supply valve 22 mounted on the first water supply pipe 21 to control water supply through the first water supply pipe 21. The first water supply pipe 21 is connected to the detergent supply device 18 so that water supplied from outside the washing machine is supplied to the detergent supply device 18. Between the detergent supply device 18 and the water tub 11 is mounted a connection pipe 23 to supply water passing through the detergent supply device 18 to the water tub 11. In this way, water passes through the detergent supply device 18 and is then supplied to the water tub 11. As a result, the detergent in the detergent supply device 18 is supplied to the water tub while being dissolved in the water. The water supply device 20 further comprises a second water supply pipe 24 to supply water to the steam generator 31, and a second water supply valve 25 mounted on the second water supply pipe 24 to control supply of water to the steam generator 31.

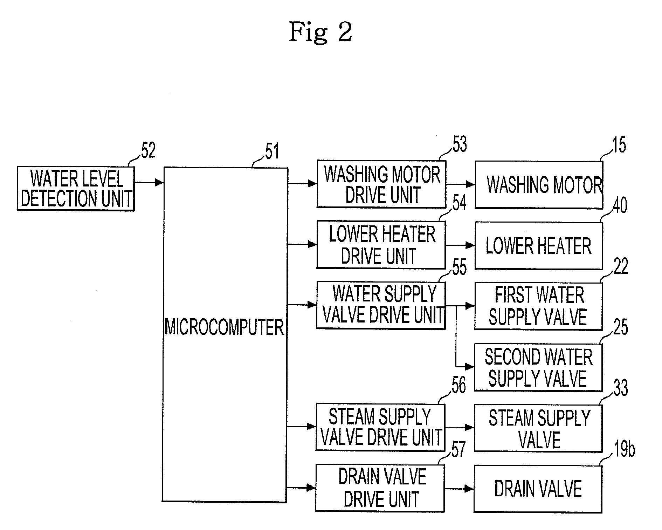

As shown in Figure 2, the drum washing machine further comprises: a water level detection unit 52 to detect a level of water supplied to the water tub; a washing motor drive unit 53 to drive the washing motor 15; a lower heater drive unit 54 to drive the lower heater 40; a water supply valve drive unit 55 to drive the first and second water supply valves 22 and 25; a steam supply valve drive unit 56 to drive the steam supply valve 33; a drain valve drive unit 57 to drive the drain valve 19b; and a microcomputer 51 to control the respective devices of the drum washing machine.

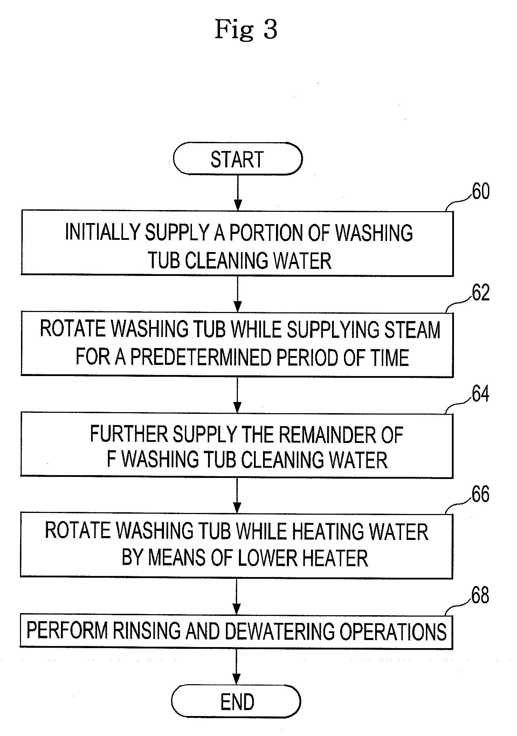

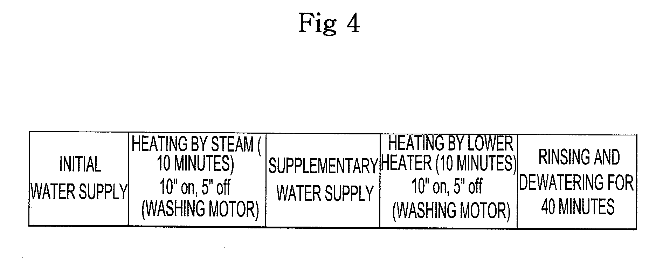

A method of cleaning the drum of the washing machine shown in Figures 1 and 2 will now be described with reference to Figures 3 and 4.

When a user selects a drum cleaning process, the first water supply valve 22 is opened to initially supply a portion of drum cleaning water, the amount of which is predetermined, to the water tub 11 through the detergent supply device 18 (step 60). During this step, it is preferable to control the amount of water supplied using a value detected by the water level detection unit 52 so that the drum fills until the water level reaches the lowest surface of the diaphragm 12 mounted at the inlet of the drum washing machine. A detergent or a disinfectant is put in the detergent supply device 18 so that water containing detergent or disinfectant dissolved therein is supplied to the water tub 11.

After the initial water supply is completed, the washing motor 15 is driven for a predetermined period of time (for example, repetitively turned on for 10 seconds and then off for 5 seconds - see Figure 4) to rotate the drum 12, and the steam generator 31 generates steam, which is supplied to the water tub 11 and the drum 12 through the steam supply pipe 34 (step 62). As the steam is supplied to the water tub 11 and the drum 12, the interior temperature of the water tub 11 is increased. Since the temperature of the steam is high, for example, above approximately 70°C, the cleaning or disinfecting activity of the detergent or the disinfectant is activated. Furthermore, when the drum 12 is cleaned with only the initially supplied water, the washing motor 15 can be operated at a relatively high rotating speed. Consequently, strong current of flow is generated by the washing motor 15, and therefore, a cleaning force is increased.

In the illustrated embodiment, the steam is supplied while the drum 12 is rotated after the water is initially supplied, although various other devices may be used to increase the interior temperature of the water tub 11. For example, a drying device disclosed in Korean Unexamined Patent Publication No. 10-2004-0022828 may be used to supply hot air to the water tub and the drum to increase the interior temperature of the water tub.

After the initial water supply and the first cleaning are completed, the first water supply valve 22 is reopened to further supply drum cleaning water to the water tub 11 (step 64). While the water is heated by the heater 40, the washing motor 15 is driven (repetitively turned on for 10 seconds and then off for 5 seconds - see Figure 4) to rotate the drum 12 (step 66). During the second cleaning, the drum is rotated while a great quantity of water is heated to clean the drum 12 and wash out various contaminants separated from the drum 12 during the first cleaning.

After the supplementary water supply and the second cleaning are completed, rinsing and spin-drying cycles are performed (step 68). In this way, the drum cleaning process is completed.

As apparent from the above description, the interior temperature of the water tub is highly increased as steam or hot air is supplied to the water tub when the drum is cleaned, and therefore, the cleaning or disinfecting activity of the detergent or the disinfectant is activated.

Also, the drum is cleaned only with a portion of the drum cleaning water during the first cleaning, and therefore, the rotating speed of the washing motor can be increased, which increases a mechanical force affecting the decontamination. Consequently, the present invention has the effect of increasing a cleaning force.

As the cleaning force is increased as described above, the drum is cleaned within a relatively short period of time. Consequently, the present invention has the effect of reducing the time necessary to clean the drum.

Although an embodiment of the present invention has been shown and described, it would be appreciated by those skilled in the art that changes may be made in this embodiment without departing from the principles of the invention, the scope of which is defined in the claims and their equivalents.

## Claims

1. A method of cleaning a rotatably mounted washing machine drum which receives laundry to be washed, **characterised by** the steps of supplying water to the drum, and rotating the drum whilst supplying heat thereto to heat the drum.

2. A method according to claim 1, wherein the method includes the step of supplying a predetermined initial amount of water to the drum and rotating the drum at a first predetermined speed whilst supplying heat to the drum before supplying an additional amount of water to the drum and rotating the drum at a second predetermined speed.

3. A method according to claim 2, wherein the second predetermined speed is faster than the first predetermined speed.

4. A method according to claim 2 or claim 3, wherein the step of supplying heat to the drum includes the step of supplying steam or hot air thereto.

5. A method according to claim 4, wherein the step of supplying heat to the drum includes the step of heating said additional amount of water using a heater prior to supplying it to the drum.

6. A washing machine comprising a rotatably mounted washing machine drum to receive laundry to be washed, means for supplying water to the drum and a heater **characterised by** a control unit configured to perform a drum cleaning cycle in which a first predetermined amount of water is supplied to the drum and the drum is rotated at a first predetermined speed before an additional predetermined amount of water is supplied to the drum and the drum is rotated at a second predetermined speed, to clean the drum.

7. A method of cleaning a drum mounted in a water tub of a washing machine, comprising supplying water necessary to clean the drum to the water tub and rotating the drum while supplying heat to the water tub by means of at least one heat supply device mounted in the washing machine.

8. The method according to claim 7 wherein the heat supply device is a steam device, and the drum is rotated while steam generated by the steam device is supplied to the water tub.

9. The method according to claim 7 wherein the heat supply device is a hot air device, and the drum is rotated while hot air generated by the hot air device is supplied to the water tub.

10. A method of cleaning a drum of a washing machine, comprising initially supplying a portion of drum cleaning water to a water tub and rotating the drum to perform first cleaning and further supplying the remainder of the drum cleaning water to the water tub and rotating the drum to perform second cleaning, wherein heat is supplied to the water tub and the drum by means of at least one heat supplying device when the first and second cleaning is performed.

11. The method according to claim 10 wherein the heat supply device is a steam device, and the drum is cleaned while the water tub and the drum are heated by steam supplied from the steam device during the first cleaning.

12. The method according to claim 10 wherein the heat supply device is a hot air device, and the drum is cleaned while the water tub and the drum are heated by hot air supplied from the hot air device during the first cleaning.

13. The method according to claim 11 wherein the heat supply device further comprises a lower heater, and the drum is cleaned while the cleaning water is heated by the lower heater during the second cleaning.

14. The method according to claim 12 wherein the heat supply device further comprises a lower heater, and the drum is cleaned while the cleaning water is heated by the lower heater during the second cleaning.

15. The method according to claim 10 further comprising performing rinsing and dewatering operations after the second cleaning is completed.

16. A washing machine comprising a water tub, a drum mounted in the water tub, at least one heat supply device to supply heat to the water tub and the drum, a water supply valve to control a flow of water to the water tub which is supplied to the drum as cleansing water and a microcomputer to perform a control operation of the water supply valve and the heat supply device, so that drum cleansing water is separately supplied several times and the drum is rotated while the heat supply device supplies heat to the water tub and the drum after each of the respective separate water supplies is completed to clean the drum.

17. The machine according to claim 16 wherein the heat supply device comprises a steam device, a hot air device or a lower heater.
